# EUROPEAN PATENT APPLICATION

(11) **EP 3 671 754 A1**
(43) Date of publication of application: **24.06.2020**
(21) Application number: 18213268.8
(22) Date of filing: 18.12.2018
(51) Int. Cl.: G16H 20/70, G06T 7/90

(54) **METHOD FOR DETERMINING A COLORATION PRODUCT RECOMMENDATION**

(71) Applicant: Henkel AG & Co. KGaA, 40589 Düsseldorf (DE)
(72) Inventor: Bonnin, Lucile, 40215 Düsseldorf (DE); Knuebel, Hans Georg, 40219 Düsseldorf (DE); Koenen, Annika, 41516 Grevenbroich (DE); Kroos, Astrid, 40789 Monheim (DE)

(57) **Abstract**

A method for determining a coloration product recommendation, in particular a hair coloration product recommendation. First images representing an object colored using a coloration product are obtained. A color of the colored object is identified on the images by determining a global color value taking into account different elements within the image. Then a desired color is obtained. A recommendation for a coloration product to achieve the desired color is determined. The recommended coloration product corresponds to the product which keeps the color difference between the desired color and the identified color below a predetermined threshold.

## Description

### TECHNICAL FIELD

The invention relates to the field of coloration compounds selection, and more particularly to ways to ensure that the appropriate coloration product is selected to change the initial color of an object into a desired target color. The invention more specifically aims to provide ways to objectivize the selection of a hair coloration product to dye hair form an initial hair color into a desired hair color.

### TECHNOLOGICAL BACKGROUND

For a person who would like to dye their hair (have their hair colored), it is important that the achieved coloring result matches the desired hair color.

Hair coloration involves the application of hair coloring agents on hair. Hair coloring agents can comprise a mixture of different dye precursors and can thus also be referred to as a coloring mixture.

While it is possible to calculate the exact color to be achieved and produced in some applied areas of color production, for example with precise photo printing with a calibrated pigment printer, doing so for hair coloration is more complicated.

A reason for this is that when coloring hair, i.e. creating a hair color, it is often the case that dyes are not used, at least not directly, but rather dye precursors. While a coloring process can consist of a multitude of different dyes, it is possible that their colorimetric properties as pure substances are not completely known.

Moreover, concentrations of dyes in the colored hair may be unknown, and it may also be unknown which concentration of the dye in the colored hair corresponds to what concentration of dye precursors in the hair coloring agent. This can be due at least in part to the fact that the combination of dye precursors interacts with each other during the formation of different dyes. To overcome these constraints, document WO2017/103050 teaches to use predictive analytics on hair data to create a model capable of predicting the color that hair coloring agents of known or unknown composition will have on hair. To build that model, hair data is produced by testing hair coloring agents on different types of hair samples (differing by their initial color, damage state or other state parameters) and measuring the color that can be achieved.

Document WO2017/103056 describes a method using predictive analytics to determine the composition of dyeing agents that could be used to achieve a desired hair color on a type of hair using a coloring process.

The model that is built using predictive analytics in the documents cited above rely on hair data that is obtained by testing the effect of each hair coloration product on different strands of hair. These measurements take time as each product is tested on different types of initial hair color or hair types (hair types can differ by their chemical or mechanical properties such as porosity, greyness levels, damage state for example). It has been shown that hair coloration products are not suitable for all hair types and hair colors. Depending on the initial hair color and more generally the initial hair state of a user, different coloration products can be recommended to achieve a same desired hair color. In order to determine an accurate match between a desired hair color and a hair coloration product that can dye a user's hair into this desired color, it is generally believed that a database of the effect each coloration product has on each existing type of hair is required. Creating this database is a costly and time consuming process.

A subjective assessment of the color that can be achieved using a coloration product, for example by relying only on the information displayed on a website or the packaging of the product is also prone to errors. Indeed, the information provided about the product and the color represented on the package is generally not a uniform color but a picture of an object (for example a user's hair) colored using this coloration product. Such pictures comprise different elements that do not display the colored object in a uniform color but rather comprise different shades and can be difficult to interpret with the eye and/or under artificial light.

An objective assessment of the true hair color that can be obtained using a hair coloration product is difficult with the naked eye based on the picture of colored hair represented on the packaging of the product. This is further due to the complex nature of hair, which can reflect and absorb light in a multitude of ways, which leads to a non-uniform color on a picture of hair colored using a coloration product. Furthermore, the subjective impression colored hair has on a user of the product is influenced by other information displayed on an image, such as the background color, the skin of the model or the presence of symbols on the packaging.

It is to be noted that similar issues arise when dealing with paints or other colored substances that are to be applied on a surface. The texture, material, shape of a surface of an object that is to be colored will influence the subjective color impression a user will have when viewing a picture of the colored object, for example on the packaging of the associated coloration product.

There is a need for an objective method for determining the coloration product that can be applied on an object (hair or any other type of surface) to change its color to a desired color, without requiring an extensive database built by testing available coloration products on a large number of possible samples.

### SUMMARY OF THE INVENTION

In order to overcome the above drawbacks, the invention provides a method for determining a coloration product recommendation from a plurality of coloration products, the method comprising:
- Obtaining a plurality of images of colored objects, each image representing an object colored using an associated coloration product from the plurality of coloration products;
- Identifying, for each image, a color of the colored object, the color corresponding to a global color value taking into account color values of elements within the image;
- Obtaining a desired color; and
- Determining a recommended associated coloration product among the plurality of coloration products, the recommended associated coloration product corresponding to an associated coloration product for which a color difference between the desired color and the identified color of the colored object is below a predetermined threshold.

The method described above circumvents the need to test the effect of coloration products on all types of objects to determine which coloration product is suitable for achieving a desired coloration result. Instead the invention relies on information provided by an image representing an object in a color achieved after application of the coloration product on the surface of the object.
The image can for example be provided on the package of the associated coloration product. It could also be obtained from another source, for example online. Based on the information provided on the image, properties of the image can be analyzed to extract an average value for the color displayed on the image. This analysis averages the color value across portions comprising different colors within the image, for example glossy parts, shadowy parts which render the selection of a reference for the color difficult.

In order to determine the coloration product that a user should select in order to change an initial color of an object (for example his hair) into a desired color, a color distance between the identified color of the colored object and the desired hair color is determined for available coloration products. It is then possible to output and recommend only those coloration products that are associated with identified colors differing by less than a predetermined threshold from the desired color. This predetermined threshold can for example be set such that noise in the image does not prevent the selection of a product that the user can further recognize as being suitable despite a mismatch with the desired color. A relative color value difference of 10% or less (when the color value is expressed in a color space for example) could for example set such a threshold.

According to an embodiment, the coloration product can be a hair coloration product, the plurality of images being images of dyed hair, each image representing hair dyed using an associated hair coloration product from a plurality of hair coloration products, wherein, for each image, a color of the dyed hair is identified, wherein the color corresponds to a global color value taking into account color values of elements within the image, wherein the desired color is a desired hair color.

The above method is particularly suitable for objectively providing appropriate hair coloration product recommendations. The effect of hair coloration compositions on hair are complex to predict and costly to reproduce on a large and representative number of samples of hair types in laboratories. A numerical analysis of the achievable color represented on an image of dyed hair simplifies the process of determining the most suitable coloration product for a user's needs.

According to an embodiment, the method may further comprise:
- Obtaining, for each associated hair coloration product, an information relating to a starting hair color of hair to which the associated hair coloration product can be applied to achieve the identified color of the dyed hair,
- obtaining an initial hair color, and
wherein determining the recommended associated hair coloration product further comprises:
- Selecting an associated hair coloration product for which the information relating to the starting hair color is compatible with the initial hair color.

Such an information may typically correspond to a middle application range, generally shown on the packaging of hair coloration products.
Further information can also be extracted from the image, for example in other parts thereof. In particular, a packaging may comprise an indication as to what types of surfaces the coloration product can be applied on. In the case of hair coloration products, packages generally indicate a middle application range corresponding to initial hair colors to which the product can be applied with a high probability of achieving the dyed hair color represented on the image of the packaging. Such information could also be extracted or deduced from analysis of other portions of the image (for example skin color, undyed portions of hair, images representing initial and achieved hair colors simultaneously).

According to an embodiment, the method may further comprise, prior to identifying a color of the dyed hair:
- Selecting a region of interest on each image, the region of interest comprising at least one hair strand void of skin and/or symbols,
the method further comprising identifying the color of the dyed hair in the selected region of interest.

Selecting a region of interest reduces the risk of adding noise into the identification of the identified color of the colored object (in particular dyed hair). The image may comprise a portion of hair, comprising mostly hair tips or hair lengths with few or no roots.

According to an embodiment, the method may further comprise:
- obtaining hair color data comprising different hair coloration compositions, each hair coloration composition being associated with at least one achieved dyed hair color, the achieved dyed hair color corresponding to a hair color measured after applying the hair coloration composition to a hair type, the hair type being associated at least with an initial hair color.

Hair color data provide a more precise and reliable information as to the effect a hair coloration product has when applied to different initial hair colors or hair types. Combining the somewhat gross information obtainable via a determination of an average color value of areas within the image (for example on the packaging) of the hair coloration product with the precise information extractable from hair data (generally obtained via laboratory measurements made on different hair types using the available products) improves the accuracy of the above method.

Furthermore, each hair coloration composition from the hair color data can be associated to a plurality of achieved dyed hair colors, each achieved dyed hair color corresponding to a hair color measured after applying the hair coloration composition to a hair type from a plurality of hair types, the hair types differing by at least one of the following : hair color, greyness levels, porosity, damage condition of hair.

The bigger the number of data available for different hair types, the more precise will the above described method be. The term "hair coloration composition" refers to the actual hair dye or mixture of dyes that makes up a hair coloration product or that could be used to make up a hair coloration product.

According to an embodiment, the method may further comprise:
- determining the recommended associated coloration composition using predictive analytics.

Predictive analytics can be generally described as a method for extracting information from large amounts of data and generating a model from said data which make it possible to also make predictions for values that are not part of the data set. Using a predictive analytics method, part of the data set can be typically used as a training data set (also referred to as a training set or training data). Based on this training data set, one or multiple models can be generated, which can be tested on the basis of data which is not part of the training data set, on the basis of the overall data, or on the basis of a specially selected part of the data.

Predictive analytics is particularly powerful when combining color information extracted from images with hair data obtained from tests conducted by applying the hair coloration product to different hair types.

According to an embodiment, each image of dyed hair can be arranged on a packaging of the associated hair coloration product.

Generally, the best region of interest is to be found on the upper lid of the packaging. However, other sources for the images can also be provided, for example a digital representation of a photograph of models having their hair dyed with the hair coloration product.

According to an embedment the elements within the image may be pixels.

According to an embedment, the identified color of the colored object can be parameterized in a color space.

This parameterization can for example be done in the L*a*b color space, or CIEL, from a scanned image of the packaging. This parameterization typically converts the format of the scanned image. The format of a scanned image can typically be in the RGB (red, green, blue) color format.

According to an embodiment, the method may further comprise:
- identifying the color of the colored object by converting colors of the image into an L*a*b color space and calculating a median value for each L, a and b parameter across pixels of the image.

Calculating a median value for color parameters across areas within the image (for example pixels) reduces the impact of noise on the identified color. Should the colored object (dyed hair) comprise glossy portions or shadowy portions, this approach reduces the influence of such graphical bias on the identified color, without totally ignoring them either, so as to convey a more balanced assessment of the achievable hair color.

According to an embodiment, the predetermined threshold may correspond to a smallest color difference obtained between the desired color and identified colors of the colored object from the plurality of images.

To determine the recommended coloration product, the method may seek to minimize the color difference between the desired color (which is then also converted into the same color space as the identified color) and the identified color.

According to an embodiment, the method may further comprise:
- identifying a text reference in the image, the text reference providing information on the color of the colored object;
- modify the global color value if the information provided by the text reference differs from the identified color of the colored object by more than a set threshold value.

According to an embodiment, at least one of the following actions may be further implemented:
- outputting an indication of a location where the recommended associated coloration product is available,
- requesting an authorization for ordering a sample of the recommended associated coloration product,
- ordering the recommended associated coloration product.

The invention also pertains to a non-transitory computer readable storage medium having stored thereon a computer program comprising instructions for execution of a method for determining a coloration product recommendation as described above.

In other words, the invention also pertains to a computer program product comprising instructions for execution of a method for determining a coloration product recommendation as described above.

### BRIEF DESRIPTION OF THE DRAWINGS

The present disclosure will hereinafter be described in conjunction with the following drawing figures, wherein like numerals denote like elements, and:
Fig. 1 shows a simplified workflow of the method according to an exemplary embodiment;
Fig. 2 shows a packaging of a hair coloration product from a perspective front view;
Fig. 3 shows a closer front view of the packaging of the hair coloration product of figure 2;
Fig. 4 shows schematic representations of non-transitory computer readable storage mediums capable of having stored thereon a computer program intended to implement the method of the invention.

### DETAILED DESCRIPTION

The following detailed description is merely exemplary in nature and is not intended to limit the disclosure or the application and uses of the subject-matter as described herein. Furthermore, there is no intention to be bound by any theory presented in the preceding background or the following detailed description.
The invention pertains to a method for determining a coloration product from a plurality of coloration products, without the need to perform extensive coloration tests on different samples. The invention instead relies on information extracted from images showing the coloration product in use or after it was applied on a surface. Such an image is typically available on the packaging of the coloration product. To avoid any bias arising from the complexity of the details illustrated on the image, the invention processes the image so as to determine a global color value taking into account color values of several elements within the image.
A "color" can be understood as an interaction of a shade (i.e. a spectral color impression, also referred to as a hue, which can be understood as what is considered the "actual color"), a color intensity (i.e. how intensively the color appears, e.g. compared with a neutral gray tone, which is also referred to as saturation, color saturation, chroma, chromaticity or depth of color) and a brightness (i.e. how light or dark the color appears).
Color information can, for example, have a parameterization in a known color space, for example in a L*a*b color space (wherein L* indicates the brightness of a color, a* the portion of green and red and b* the portion of blue and yellow of the color, where the abbreviated form Lab and/or individual L, a and/or b are used here) in an RGB color space with color portions in red, green and blue, in a CMYK color space with color portions in cyan, magenta, yellow and black or in any other arbitrary color space.
The term "shade" can be understood to mean the spectral color impression of a color independently of how it can be parameterized, such as a point in a two-dimensional color space (e.g. a*b* of the L*a*b* system) or a ratio of color portions (such as in the RGB color space or in the CMYK color space).
In various exemplary embodiments, a color space from which the color information (e.g. the hair color information of the colored hair or the hair before the coloring, which is also referred to as the initial hair color) arose, or in which the color information is represented (for example, if a hair color is represented, see below) can be procured so that a determined or represented color is independent of a medium through which the color is determined or represented (e.g. color measuring device, screen, printer, scanner, human eye, etc.). The color space can be, for example, an L*a*b* color space and the color information can, for example, be a shade parameterized by employing a* and b*. The uniform representation in the medium-independent color space can make it possible, for example, to present a close-to-reality coloring result to be expected, for example, in which the same color impression of a color achieved by coloring is left on the observer in a representation of the result to be expected, for example as printing on a package, an advertisement on a computer screen, etc.

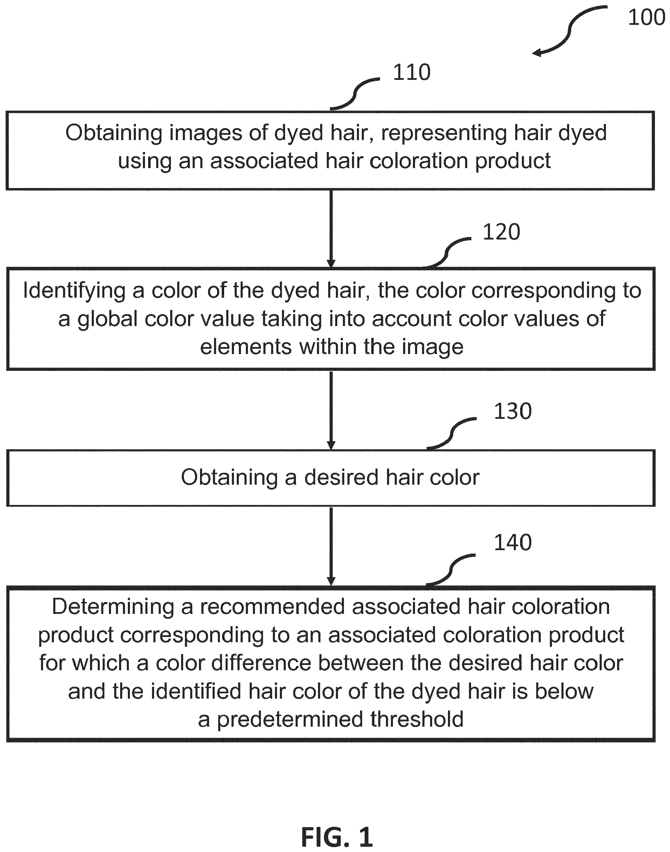

Figure 1 provides a flowchart summarizing four of the steps that form part of a method 100 for determining a recommended coloration product. Figure 1 mentions explicitly the use of the method to determine recommended hair coloration products but the same logic applies to other applications. For example, the method could equally be used for selecting other coloration means such as paints for skin, teeth, or any other surface.
First, the method consists in obtaining images of hair dyed using a hair coloration product. Each image is associated with a different hair coloration product.
This step of obtaining 110 a plurality of images can consist in receiving a digital picture representing the dyed hair in any form. For example, the dyed hair could be seen on a photograph of a model whose hair was dyed using the associated hair coloration product. Alternatively, the color could be represented with a simulated uniform or non uniform color showing the appearance hair dyed with this hair coloration product would have. In most cases, such a representation would not be a uniform color block but rather a representation of a strand of hair, comprising shiny or glossy parts and darker parts.
This digital picture could be stored in any known format for storing pictures and accessible via a computer for example. In such a case, the digital image can first be converted into a format which represents color information in a color space such as CIEL, Lab or RGB (R standing for red, G for green and B for blue) for pixels sharing the same color or elements of any other size sharing the same color.
The images of dyed hair could also be provided as printed material, for example on a packaging of the associated hair coloration product. In that case, the images could be scanned using for example a flatbed scanner calibrated with an IT8-target using appropriate software. A wide color space such as RGB, CIEL, CMYK, Lab or eciRGB (for European color initiative RGB) is preferably used to numerically store the scanned image.
The image could typically be on the top lid of the packaging of a hair coloration product, which generally represents strands of hair.

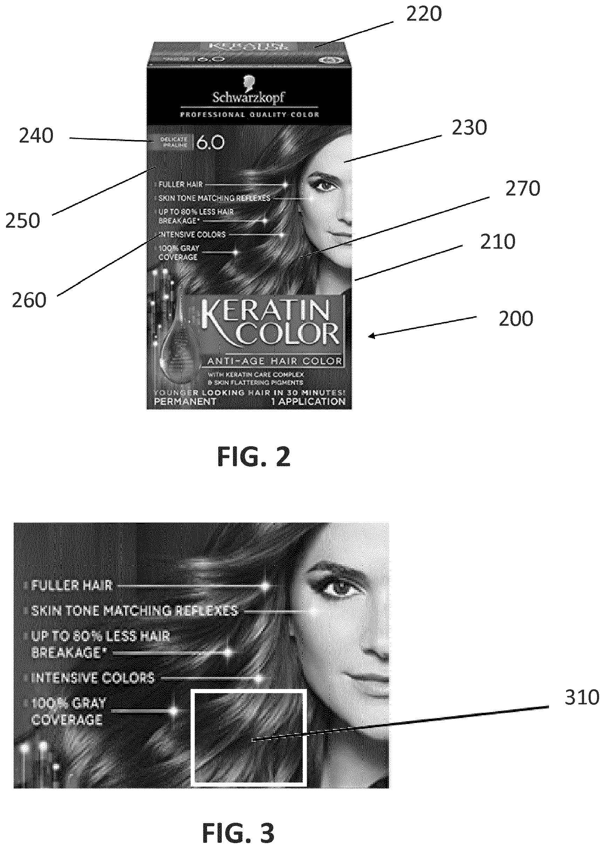

Figure 2 shows one example of a packaging 200 of a hair coloration product, comprising a lid 220 and a front label 210. The front label 210 further comprises the picture of a model on a dark background 250, a text reference 240 describing and identifying the color that can be achieved using the coloration product, skin 230 of the model, text and symbols 260 and colored hair 270, comprising glossy and darker areas.
The method further processes these images by identifying 120, for each image, a color of the dyed hair.
To do so, the image may first be processed by selecting a region of interest which comprises no skin 230, scalp, clothes or symbols 260 such as text.
Alternatively, the region of interest may be selected in a gross manner and post processed to remove all undesired elements therefrom.
Figure 3 provides one example of such a region of interest 310 indicated with a white square on the front label 210 of the hair coloration product packaging of figure 2.
As can be seen on figure 3, the region of interest is not a block comprising a uniform color. It comprises different glossy zones and shadowy zones which render the selection of a reference point to be considered as representing the color achievable with the coloration product as unreliable. To overcome this, the invention identifies a global color value which takes into account several different colored elements on the image in order to avoid any bias in the selection of the image or the region of interest 310.

This identification can be done by converting the format of the image into a Lab color space using suitable software. The three channels L a and b are then separated for each element of the image and processed independently. The elements in the picture can be pixels, areas of uniform color on the picture or features identified on the image (for example tips, roots, lengths of hair, glossy areas on a hair strand, darker areas on a hair strand).
In case no region of interest 310 was selected, or to avoid any error in this selection, the image is advantageously processed to exclude symbols 260 from the image as well as elements that are not associated with hair, such as skin, scalp or clothing for example.
The global color value which takes into account several elements within the image can be determined in the color space by taking the median value for each L, a and b channel across all elements within the image or the region of interest 310. Advantageously, these elements would be pixels, so that each pixel contributes equally to the global color value that is determined using this approach.
Advantageously, the image would not contain a majority (more than 50% of all pixels on the image) of features of one type such as glossy or dark areas. When one type of feature clearly dominates in the image, the identified color of dyed hair might be incorrectly estimated. As long as no feature associated to a visual effect such as glossy reflection or shadowy areas in the image represents more than 50% of all pixels, the identified color of dyed hair, identified using the above method, conveys a balanced assessment of the color achievable using the associated hair coloration product.
It is to be noted that other color identification means can be applied. For example, instead of a median value, it is also possible to determine an average value for each channel in the color space.

Then the method continues by obtaining 130 a desired color. This desired color can be input by a user via a man-machine interface on a mobile device, a computer, a tablet either in written form or vocally. It is possible to select a desired color by selecting one from a range of achievable colors based on the existing coloration products available. It is also possible to input a desired color that is not achievable with existing coloration products, in order to determine a coloration product that provides a closest match with the desired color.
Finally, the method further proceeds by determining 140 a recommended coloration product among available coloration products. To do so, the identified color of the colored object is compared with the desired color. For that purpose, both colors are converted into the same color space. Advantageously, the desired color is converted into the color space in which the identified color is expressed. Then, a color difference is measured between the identified color and the desired color.
One possibility is to output, as a recommended coloration product, the coloration product that is associated with the identified color that differs by the smallest amount from the desired color. In that case, color differences are measured between the desired color and available identified colors.
However, it is also possible to set a threshold below which the identified color is considered as being close enough to the desired color so that the associated coloration product would still provide an acceptable color to a user. In that case, it is possible not to measure color differences between the desired color and all available identified colors. Calculation of color differences can be stopped if for at least one identified color a color difference below the set threshold is found.
Furthermore, when a threshold is set for the acceptable color difference, it is possible to recommend more than one coloration product which gives the user a larger choice of products. The threshold can be used to take into account the possibility that the identified colors do not accurately reflect the true color of the coloration products once they are applied. Indeed, the color achievable with a coloration product (the identified color) is extracted from an image. There is some noise in that image, due for example to the conditions in which the image was taken, or the nature of the object on which the image was printed (generally cardboard paper for packaging), or due to noise introduced by the scanner or camera that was used to analyze the image. In that case, the identified color that has the smallest color difference with the desired color may not necessarily be associated with the best coloration product for a user's needs.
It is for example possible to set a threshold value for the color difference as being a value equal to or lower than 10% of the value of the identified or desired color, across each channel in the color space in which these colors are expressed. It is also possible to fine tune this percentage and define a different percentage for each channel in the color space.
To calculate the color difference, it is possible to calculate a value corresponding to the difference of the color values for each channel in the color space in which the colors are expressed (for example, the L, a and b channels of the Lab color space).
It is possible to set the weight given to each channel when determining the global color difference. For example, the best color match between the identified color and the desired color could be considered as having the lowest value difference along the a and b channels, regardless of the difference along the L channel. Specific weight could be given to each channel to fine tune this threshold and criterion for determining the color differences. Based on these color differences, it is also possible to rank the coloration products from the one associated with the smallest color difference to the one associated with the largest color difference with the desired color. That way, a user can see the output of the method in the form of ranked recommended products, the displayed products comprising those that correspond to color differences below the predetermined threshold.
It is also possible to determine the color difference by giving an equal weight to the value difference across all channels.

In a further embodiment, the method may further take into account an information relating to a starting hair color of hair to which each hair coloration product may be applied. Indeed. The initial hair color of a user limits the range of coloration products that are compatible with this color to achieve a desired hair color. For example, it would be easier to dye light brown hair into a blond color than black hair.
Most hair coloration products provide information relating to the middle application range, that is to say, the range of starting hair colors to which the hair coloration product can be applied with a reasonable likelihood of achieving the hair color represented on the packaging. This middle application range can be expressed either in words, or with a picture showing the color of the starting hair to which the coloration product can be applied.
The above method can be further used to scan such a portion of the packaging and identify the colors corresponding to the middle application range. Otherwise, the color reference mentioned on the package can be converted into a color value expressed in a color space.
The method can further obtain an initial hair color, from a user. This hair color can be provided by the user himself (being his own hair color or someone else's hair color if he intends to find a suitable coloration product for another person). It can also be provided by someone else, such as a hair styling professional. Alternatively, the initial hair color can be determined using a measuring device, for example an analyzer that uses optical means to determine the hair color and hair state in a more objective way.
The initial hair color can further be input manually via a man-machine interface, on a mobile device, online or via terminal. The information can also be provided vocally by naming the initial hair color, or selected from a terminal which displays a plurality of possible starting hair colors to choose from.
The initial hair color can be identified as described above and compared to the middle application range provided on the packaging by measuring differences in color values as described above. That way the recommended hair coloration products can be limited only to those that are associated with a middle application range comprising the initial hair color. The above method does not require a database of laboratory test results testing each hair coloration product on each type of hair and initial hair color. However, the method can further combine information extracted from such laboratory tests if they are available to improve the accuracy of the determination of the recommended hair coloration product.
When such information from laboratory tests is available, it is possible to combine the information extracted from images with information extracted from laboratory tests.
In such a case, it is further possible to determine two types of recommended coloration products. A first type of recommended coloration product may be determined among coloration products for which laboratory tests were conducted to test the effect of the coloration product on different initial hair colors. A second type of recommended coloration products may be determined using the method described above.
In such a situation, the first type of recommended hair coloration products could be ranked better than the second type of recommended hair coloration products.
The identification of the first type of recommended hair coloration products can be further enhanced using predictive analytics. Indeed, it is possible that the hair data available via the laboratory test results do not comprise the initial hair color provided by a user and/or his desired hair color. In that case, predictive analytics provides a powerful means to extract a model of correspondence between initial hair colors and achievable hair colors using different types of coloration products. The model can predict a correspondence between initial hair colors and desired hair colors even for desired hair colors that are not associated with any existing hair coloration product. That way, the model can further allow identification of a recommended hair coloration product that provides a closest match with the initial and desired hair color. Further examples of how such a model is built can be found in the following applications: WO2017/103050 and WO2017/103056.
The predictive analytics model can further be constructed by taking other hair properties into account (which can also be obtained from a user, or analyzed on a user's hair using an analyzing device as explained above). These hair properties can for example include fastness to washing, light fastness, grayness, hair damage state, porosity.

According to a further embodiment, it is also possible to extract color information from text provided for example on the packaging 200 of the hair coloration product. Text references 240 can name a color achievable using the hair coloration product within the packaging. This information can be converted into the same color space as the one in which the identified color of the dyed hair is expressed to check whether the identification of the color was successful. In case the offset between the identified color and the one extracted from the text reference 240 is larger than a set value (for example a 10% difference across all channels in the color space), then the identified color is corrected. This correction can for example be implemented by choosing a different region of interest 310, or calibrating the scanner used to convert the images into a digital picture.

The invention may further comprise ordering the recommended hair coloration product automatically, or after confirmation received from a request sent to a user, or via an entry (for example typed or dictated) received from the user.
The method may also output a location where the recommended hair coloration product is available.
The steps of the examples and embodiments described above can be implemented by a processor such as a computer. A computer program product comprising steps of the above-described method can be used to implement the method on a computer.

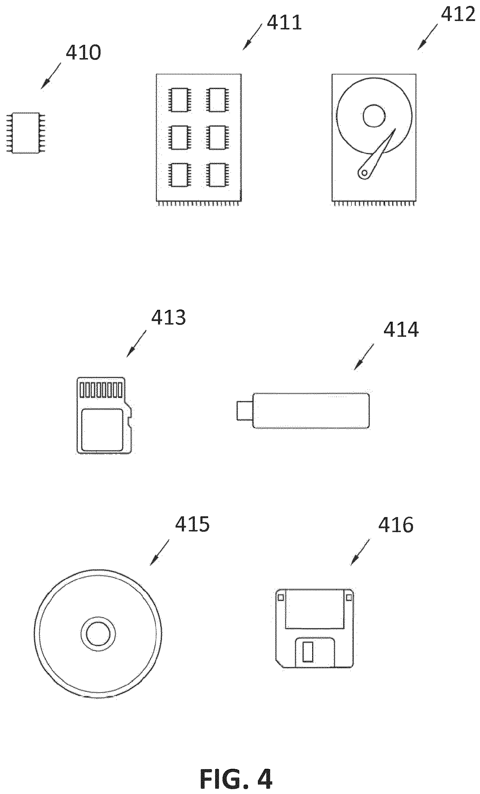

Figure 4 provides examples of non-transitory computer readable storage mediums on which a computer program comprising instructions to implement the method of the invention can be stored. These could for example comprise a processor or chip 410, an electronic circuit comprising several processors or chips 411, a hard drive 412, a flash or SD card 413, a USB stick 414, a CD-ROM or DVD-ROM or Blue-Ray disc 415, or a diskette or floppy disk 416.
While at least one exemplary embodiment has been presented in the foregoing detailed description, it should be appreciated that a vast number of variations exist. It should also be appreciated that the exemplary embodiment or exemplary embodiments are only examples, and are not intended to limit the scope, applicability, or configuration of the various embodiments in any way. Rather, the foregoing detailed description will provide those skilled in the art with a convenient road map for implementing an exemplary embodiment as contemplated herein. It being understood that various changes may be made in the function and arrangement of elements described in an exemplary embodiment without departing from the scope of the various embodiments as set forth in the appended claims.

## Claims

1. A method (100) for determining a coloration product recommendation from a plurality of coloration products, the method comprising:
- Obtaining (110) a plurality of images of colored objects, each image representing an object colored using an associated coloration product from the plurality of coloration products;
- Identifying (120), for each image, a color of the colored object, the color corresponding to a global color value taking into account color values of elements within the image;
- Obtaining (130) a desired color; and
- Determining (140) a recommended associated coloration product among the plurality of coloration products, the recommended associated coloration product corresponding to an associated coloration product for which a color difference between the desired color and the identified color of the colored object is below a predetermined threshold.

2. The method of claim 1, wherein the coloration product is a hair coloration product, the plurality of images being images of dyed hair, each image representing hair dyed using an associated hair coloration product from a plurality of hair coloration products, wherein, for each image, a color of the dyed hair is identified, wherein the color corresponds to a global color value taking into account color values of elements within the image, wherein the desired color is a desired hair color.

3. The method according to claim 2, further comprising:
- Obtaining, for each associated hair coloration product, an information relating to a starting hair color of hair to which the associated hair coloration product can be applied to achieve the identified color of the dyed hair;
- Obtaining an initial hair color;
wherein determining the recommended associated hair coloration product further comprises:
- Selecting an associated hair coloration product for which the information relating to the starting hair color is compatible with the initial hair color.

4. The method according to any one of claims 2 to 3, further comprising, prior to identifying a color of the dyed hair:
- Selecting a region of interest (310) on each image, the region of interest comprising at least one hair strand void of skin and/or symbols,
the method further comprising identifying the color of the dyed hair in the selected region of interest.

5. The method according to any one of claims 2 to 3, further comprising:
- obtaining hair color data comprising different hair coloration compositions, each hair coloration composition being associated to at least one achieved dyed hair color, the achieved dyed hair color corresponding to a hair color measured after applying the hair coloration composition to a hair type, the hair type being associated at least to an initial hair color.

6. The method of claim 5 wherein each hair coloration composition from the hair color data is associated to a plurality of achieved dyed hair colors, each achieved dyed hair color corresponding to a hair color measured after applying the hair coloration composition to a hair type from a plurality of hair types, the hair types differing by at least one of the following : hair color, greyness levels, porosity, damage condition of hair.

7. The method according to any one of claims 5 or 6, further comprising:
- determining the recommended associated coloration composition using predictive analytics.

8. The method according to any one of the preceding claims, wherein each image is arranged on a packaging (200) of the associated coloration product.

9. The method according to any one of the preceding claims, wherein the elements within the image are pixels.

10. The method according to any one of the preceding claims, wherein the identified color of the colored object is parameterized in a color space.

11. The method according to any one of the preceding claims, further comprising:
- identifying the color of the colored object by converting colors of the image into an L*a*b color space and calculating a median value for each L, a and b parameter across pixels of the image.

12. The method according to any one of the preceding claims, wherein the predetermined threshold corresponds to a smallest color difference obtained between the desired color and identified colors of the colored object from the plurality of images.

13. The method according to any one of the preceding claims, further comprising:
- identifying a text reference (240) in the image, the text reference providing information on the color of the colored object;
- modify the global color value if the information provided by the text reference differs from the identified color of the colored object by more than a set threshold value.

14. The method according to any one of the preceding claims, wherein at least one of the following actions are further implemented:
- outputting an indication of a location where the recommended associated coloration product is available,
- requesting an authorization for ordering a sample of the recommended associated coloration product,
- ordering the recommended associated coloration product.

15. A non-transitory computer readable storage medium (411-415) having stored thereon a computer program comprising instructions for execution of a method for determining a coloration product recommendation according to any one of the preceding claims.
